# EUROPÄISCHE PATENTSCHRIFT

(11) **EP 2 263 574 B1**
(45) Veröffentlichungstag und Bekanntmachung des Hinweises auf die Patenterteilung: **07.09.2011**
(21) Anmeldenummer: 10006012.8
(22) Anmeldetag: 10.06.2010
(51) Int. Cl.: A61B 17/28, A61B 17/29, A61B 17/00

(54) **Medizinisches Instrument**
Medical instrument
Instrument médical

(30) Priorität: 17.06.2009 DE 102009025663
(43) Veröffentlichungstag der Anmeldung: 22.12.2010
(73) Patentinhaber: Karl Storz GmbH & Co. KG, 78532 Tuttlingen (DE)
(72) Erfinder: Schneider, Sven, 78532 Tuttlingen (DE)
(74) Vertreter: Straub, Bernd

(56) Entgegenhaltungen:
- DE-U1-202005 020 819
- US-A- 5 234 460
- US-A1- 2007 016 248

## Beschreibung

Die Erfindung betrifft ein medizinisches Instrument mit einem Schaft, an dessen distalem Ende ein Werkzeug angeordnet ist und an dessen proximalem Ende eine aus mindestens zwei Griffteilen bestehende Handhabe angeordnet ist, wobei das Werkzeug über ein verstellbar ausgebildetes Griffteil der Handhabe betätigbar ist und wobei mindestens ein Griffteil der Handhabe aus einem starren Teilbereich sowie einem um den starren Teilbereich verschwenkbaren Teilbereich besteht.

Ein medizinisches Instrument gemäß der Oberbegriff des Anspruch 1 ist beispielsweise aus der DE 10 2005 027 418 A1 bekannt. Durch die Ausbildung eines Teilbereichs eines Handgriffs als um die Längsachse des Griffteils verschwenkbaren Teilbereich ist es bei diesem bekannten Instrument möglich, ohne Loslassen oder Umgreifen der Handhabe die Lage der Handhabe in der Hand einerseits so zu variieren, dass keine Druckstellen an der Hand des Operateurs entstehen und andererseits eine stets optimale Lage der Griffteile zur Hand- und Fingerstellung des Operateurs gewährleistet ist, um die bestmögliche Krafteinleitung über die Hand auf das Instrument zu ermöglichen.

Diese Art der verschwenkbaren Ausgestaltung eines Griffteils der Handhabe hat sich in der Praxis durchaus bewährt, jedoch ist der Montage- und Demontageaufwand sehr hoch, um den verschwenkbaren Teilbereich des Griffteils zu Reinigungszwecken vom starren Griffteil zu lösen bzw. wieder mit diesem zu verbinden.

Davon ausgehend liegt der Erfindung die **Aufgabe** zugrunde, ein medizinisches Instrument zu schaffen, das bei einfachem Aufbau schnell und gründlich zu reinigen ist.

Die **Lösung** dieser Aufgabenstellung ist erfindungsgemäß dadurch gekennzeichnet, dass der verschwenkbare Teilbereich des Griffteils über einen Rastmechanismus abnehmbar am starren Teilbereich des Griffteils festlegbar ist und dass der Rastmechanismus einen Rasthaken aufweist der über einen Druckknopf betätigbar ausgebildet ist.

Durch die erfindungsgemäße Ausbildung eines Rastmechanismus zur Montage und Demontage des verschwenkbaren Teilbereichs ist es ohne Werkzeugeinsatz einfach und schnell möglich, den verschwenkbaren Teilbereich insbesondere zu Reinigungszwecken abzunehmen und wieder am starren Teilbereich festzulegen.

Gemäß einer praktischen Ausführungsform der Erfindung wird vorgeschlagen, dass der Rastmechanismus einen an einem der miteinander zu verrastenden Bauteile angeordneten, mit einem Rasthaken versehenen federbelasteten Druckknopf und einen an dem anderen Bauteil angeordneten, mit dem Rasthaken korrespondierenden Verriegelungselement, insbesondere einen Verriegelungsstift aufweist. Diese Ausbildung des Rastmechanismus als Rasthaken und korrespondierendes Verriegelungselement, insbesondere eines Verriegelungsstifts stellt eine besonders einfach zu fertigende und zu handhabende Ausgestaltungsform des erfindungsgemäßen Rastmechanismus dar.

Mit einer bevorzugten Ausführungsform der Erfindung wird vorgeschlagen, dass der Rastmechanismus eine am verschwenkbaren Teilbereich festlegbare Gleit- und Spannbuchse umfasst, die als proximales Drehlager für den verschwenkbaren Teilbereich dient. Durch die Verwendung der Gleit- und Spannbuchse werden die Funktionen Verrasten der Bauteile starrer Teilbereich und verschwenkbarer Teilbereich sowie die verschwenkbewegliche Lagerung des verschwenkbaren Teilbereichs in einer Baugruppe zusammengefasst. Gemäß einer praktischen Ausführungsform wird vorgeschlagen, dass der Druckknopf an der Gleit-und Spannbuchse gelagert ist.

Um einerseits ein leichtgängiges Verschwenken des verschwenkbaren Teilbereichs um den starren Teilbereich zu ermöglichen und andererseits den Reibwiderstand individuell einstellen zu können, wird mit der Erfindung weiterhin vorgeschlagen, dass die Gleit- und Spannbuchse ein verdrehfest mit dem Druckknopf verbundenes Drehlager sowie eine mit dem Drehlager zusammenwirkende verdrehfest mit dem verschwenkbaren Teilbereich verbundene Gleitbuchse umfasst.

Zur Erzeugung einer möglichst verschleißarmen Drehlagerung wird mit der Erfindung vorgeschlagen, dass zumindest die Kontaktflächen des Drehlagers und der Gleitbuchse aus einem gleitfähigen Material bestehen. Alternativ zu der Ausbildung nur der Kontaktflächen aus einem gleitfähigen Material ist es selbstverständlich auch möglich, das Drehlager und die Gleitbuchse vollständig aus einem gleitfähigen Material, wie beispielsweise Teflon ®, zu fertigen oder aber die Kontaktflächen mit dem gleitfähigen Material zu beschichten.

Die Kontaktflächen des Drehlagers und der Gleitbuchse sind vorteilhafterweise miteinander korrespondierend konisch ausgebildet, um einen festen Sitz der beiden Bauteile aufeinander bzw. ineinander zu gewährleisten.

Zur Einstellung des Reibwerts zwischen dem Drehlager und der Gleitbuchse wird erfindungsgemäß vorgeschlagen, dass das Drehlager und die Gleitbuchse über ein Spannelement gegeneinander verspannbar sind, wobei das Spannelement gemäß einer ersten erfindungsgemäßen Ausführungsform als Federelement ausgebildet ist.

Gemäß einer alternativen Ausführungsform zur Ausbildung des Spannelements wird erfindungsgemäß vorgeschlagen, dass das Spannelement als Spannschraube, insbesondere als Madenschraube, ausgebildet ist.

Weiterhin wird mit der Erfindung vorgeschlagen, dass am starren Teilbereich und/oder am verschwenkbaren Teilbereich des Griffteils Anlaufschrägen als Zentrierhilfen ausgebildet sind. Diese Anlaufschrägen ermöglichen einerseits ein klemmfreies Aufeinanderschieben der miteinander zu verbindenden Bauteile und andererseits auch eine Zentrierung der Bauteile in der richtigen Lage zueinander, so dass der verschwenkbare Teilbereich des Griffteils auch in der verschwenkten Stellung des Griffteils auf den starren Teilbereich des Griffteils aufschiebbar ist.

Schließlich wird mit der Erfindung vorgeschlagen, dass der Verschwenkwinkel des verschwenbaren Teilbereichs durch die bezüglich der Längsachse des Griffteils exzentrische Lagerung des verschwenkbaren Teilbereichs am starren Teilbereich begrenzt ist. Die Begrenzung des Verschwenkwinkels des verschwenkbaren Teilbereichs des Griffteils ist vorteilhaft, um ein Verrutschen des Griffteils in der Hand des Operateurs zu verhindern.

Weitere Merkmale und Vorteile der Erfindung ergeben sich anhand der zugehörigen Zeichnungen, in denen ein Ausführungsbeispiel eines erfindungsgemäßen medizinischen Instruments nur beispielhaft dargestellt ist. In der Zeichnung zeigt:
- Fig. 1: eine Seitenansicht eines erfindungsgemäßen medizinischen Instruments in ei- ner ersten Arbeitsstellung der Griffteile;
- Fig. 2: eine Rückansicht der Darstellung gemäß Fig. 1, jedoch ein Griffteil in einer verschwenkten Arbeitsstellung darstellend;
- Fig. 3: eine geschnittene Seitenansicht des den verschwenkbaren Teilbereich aufwei- senden Griffteils und
- Fig. 4: eine Explosionszeichnung des Rastmechanismus mit Gleit-und Spannbuchse.

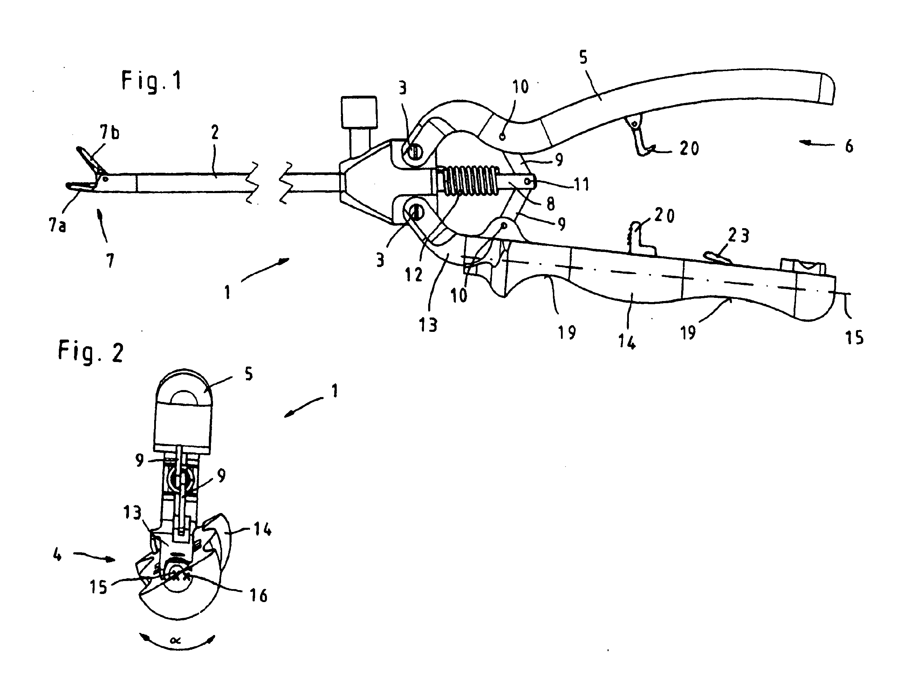

Die Abbildung Fig. 1 zeigt die Seitenansichten eines als Greif- und/oder Schneidinstrument ausgebildeten medizinischen Instruments 1, dessen Kraftübertragungsmechanismus vielseitig verwendet werden kann, wie beispielsweise für Stanzen, Scheren, Nadelhalter, Fassinstrumente und dergleichen.

Das dargestellte medizinische Instrument 1 besteht im Wesentlichen aus einem hohlen Schaft 2, an dessen proximalern Ende eine aus zwei, um jeweils zugehörige Schwenkachsen 3 verschwenkbaren Griffteilen 4 und 5 bestehende Handhabe 6 angeordnet ist. Am distalen Ende des Schaftes 2 ist ein Werkzeug 7 angeordnet, welches beim dargestellten Ausführungsbeispiel aus einem starr mit dem Schaft 2 verbundenen Maulteil 7a sowie einem verschwenkbaren Maulteil 7b besteht. Zum Öffnen und Schließen der Maulteile 7a und 7b des Werkzeugs 7 über die Betätigung der verschwenkbaren Griffteile 4 und 5 der Handhabe 6 stehen die Griffteile 4, 5 und das verschwenkbare Maulteil 7b über eine Zug-/Druckstange 8 miteinander in Wirkverbindung.

Wie aus der Abbildung Fig. 1 weiterhin ersichtlich, erfolgt die Kopplung der Griffteile 4 und 5 mit der Zug-/Druckstange 8 bei der dargestellten Ausführungsform über zwischengeschaltete Schwenkhebel 9, die mit einem freien Ende verschwenkbar an einem Lagerpunkt 10 an jeweils einem der Griffteile 4, 5 und mit dem anderen freien Ende an einer gemeinsamen Schwenkachse 11 an der Zug-/Druckstange 8 gelagert sind. Die Anlenkung der Griffteile 4 und 5 über die Schwenkhebel 9 an der Zug-/Druckstange 8 ist derart ausgelegt, dass beim Zusammendrücken der Griffteile 4, 5 die Zug-/Druckstange 8 über die Schwenkhebel 9 zum proximalen Ende des medizinischen Instruments 1 gezogen wird, was wiederum ein Verschwenken des verschwenkbaren Maulteils 7b des Werkzeugs 7 in die geschlossene Stellung bewirkt.

Zum Öffnen der Maulteile 7a, 7b des Werkzeugs 7 bedarf es somit des Auseinanderbewegens der Griffteile 4, 5 der Handhabe 6, wodurch die Zug-/Druckstange 8 über die Schwenkhebel 9 zum distalen Ende des medizinischen Instruments 1 gedrückt wird. Um das Auseinanderdrücken der Griffteile 4, 5 der Handhabe 6 zu erleichtern, ist auf der Zug-/Druckstange 8 ein als Zugfeder ausgebildetes Federelement 12 angeordnet, über das die Griffteile 4, 5 in die geöffnete Stellung vorgespannt sind.

Um dem Operateur zu ermöglichen, auch ohne Loslassen oder neues Ergreifen der Griffteile 4 und 5 der Handhabe 6 die Lage der Griffteile 4 und 5 in der Hand zu verändern, ist der Griffteil 4 zweiteilig aufgebaut, nämlich bestehend aus einem starren Teilbereich 13 sowie einem relativ zum starren Teilbereich 13 verschwenkbaren Teilbereich 14, wie dies insbesondere der Abbildung Fig. 2 zu entnehmen ist. Bei der dargestellten Ausführungsform ist der verstellbare Teilbereich 14 um eine exzentrisch zur Längsachse 15 des Griffteils 4 angeordnete Schwenkachse 16 verschwenkbar an dem starren Teilbereich 13 des Griffteils 4 gelagert, wobei die verschwenkbare Lagerung des verschwenkbaren Teilbereichs 14 über ein am starren Teilbereich 13 angeformten distalen Lagerzapfen 17 sowie ein proximales Drehlager 18 erfolgt.

Durch die exzentrische Anordnung der Schwenkachse 16 bezüglich der Längsachse 15 des Griffteils 4 ergibt sich automatisch eine Begrenzung des Verschwenkwinkels α, um den der verschwenkbare Teilbereich 14 um den starren Teilbereich 13 des Griffteils 4 verschwenkbar ist, da diese Exzentrizität ab einem konstruktiv einstellbaren Verschwenkwinkel α ein klemmendes Anlaufen des verschwenkbaren Teilbereichs 14 am starren Griffteil 13 zur Folge hat.

Die Abbildung Fig. 1 zeigt weiterhin, dass am verschwenkbaren Teilbereich 14 des Griffteils 4 Griffmulden 19 zur Aufnahme der Finger der Haltehand ausgeformt sind, wodurch das Ergreifen und Verschwenken des verschwenkbaren Teilbereichs 14 erleichtert wird. Weiterhin ist ein Arretiermechanismus 20 vorgesehen, über den die Griffteile 4 und 5 der Handhabe 6 in der zusammengedrückten Stellung der Griffteile 4 und 5, also in der geschlossenen Stellung des Werkzeugs 7, aneinander festlegbar sind, um den Operateur zu entlasten.

Da sich Verunreinigungen zwischen dem starren Teilbereich 13 und dem verschwenkbaren Teilbereich 14 des Griffteils 4 ablagern können, ist es erforderlich, die beiden Teilbereiche 13 und 14 des Griffteils 4 zu Reinigungszwecken voneinander trennen zu können. Zu diesem Zweck ist der verschwenkbare Teilbereich 14 des Griffteils 4 über einen Rastmechanismus 21 abnehmbar bzw. auswechselbar am starren Teilbereich 13 des Griffteils 4 festlegbar.

Zusätzlich zur Möglichkeit, den verschwenkbaren Teilbereich 14 und den starren Teilbereich 13 des Griffteils 4 einfach und gründlich reinigen zu können, bietet die abnehmbare bzw. auswechselbare Lagerung des verschwenkbaren Teilbereichs 14 die Möglichkeit, einen in Größe und/oder Form anderen verschwenkbaren Teilbereich 14 am starren Teilbereich 13 des Griffteils 4 festzulegen, wenn dies für den Einsatzzweck erforderlich und/oder vom Operateur gewünscht ist.

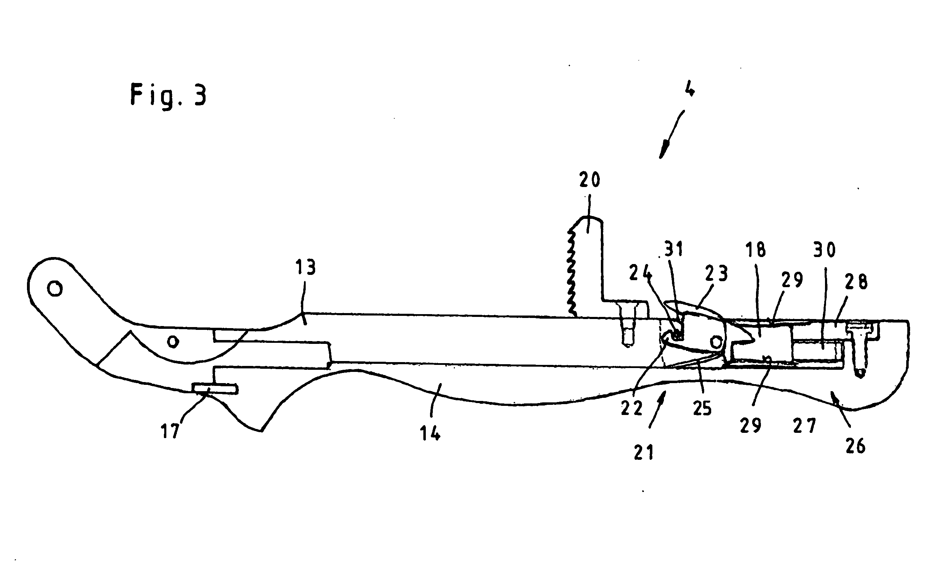

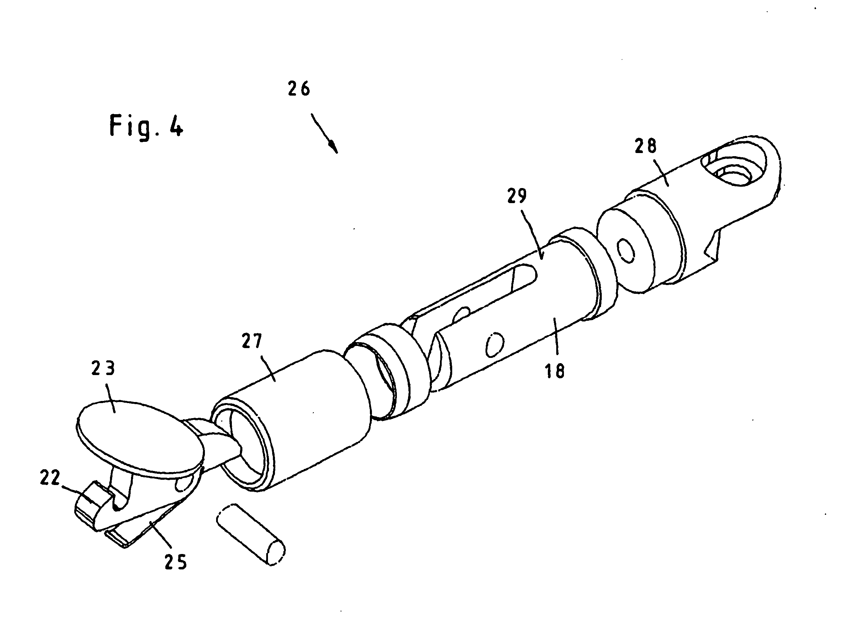

Der Aufbau und die Handhabung des Rastmechanismus 21 ergibt sich aus den Abbildungen Fig. 3 und 4. Bei der dargestellten Ausführungsform besteht der Rastmechanismus 21 aus einem am verschwenkbaren Teilbereich 14 des Griffteils 4 angeordneten, mit einem Rasthaken 22 versehenen federbelasteten Druckknopf 23 und einem am starren Teilbereich 13 des Griffteils 4 angeordneten, mit dem Rasthaken 22 korrespondierenden Verriegelungsstift 24. Als Federelement zum Überführen des Rasthakens 22 in eine den Verriegelungsstift 24 hintergreifende Raststellung dient bei der dargestellten Ausführungsform des Rastmechanismus 21 eine am Druckknopf 23 festgelegte Blattfeder 25.

Alternativ zu der dargestellten Ausführungsform ist es auch möglich, den mit dem Rasthaken 22 versehenen Druckknopf 23 am starren Teilbereich 13 und den Verriegelungsstift 24 am verschwenkbaren Teilbereich 14 des Griffteils 4 anzuordnen und/der das mit dem Rasthaken 22 versehene Bauteil nicht als Druckknopf, sondern als Schieber oder Hebel auszubilden.

Wie weiterhin aus Fig. 3 und 4 ersichtlich, ist am Druckknopf 23 eine Gleit- und Spannbuchse 26 gelagert, die aus dem verdrehfest mit dem Druckknopf 23 verbundenen Drehlager 18, einer verdrehfest mit dem verschwenkbaren Teilbereich 14 verbundenen und mit dem Drehlager 18 zusammenwirkenden Gleitbuchse 27 besteht sowie einem am verschwenkbaren Teilbereich 14 festlegbaren Spannteil 28 besteht. Das Drehlager 18 bildet bei dieser Konstruktion die proximalseitige Lagerung des verschwenkbaren Teilbereichs 14 des Griffteils 4.

Zur Erzeugung einer möglichst verschleißarmen Drehlagerung bestehen die Kontaktflächen 29 des Drehlagers 18 und der Gleitbuchse 27 vorteilhafterweise aus einem gleitfähigen Material. Alternativ zu der Ausbildung nur der Kontaktflächen 29 aus einem gleitfähigen Material ist es auch möglich, das Drehlager 18 und die Gleitbuchse 27 vollständig aus einem gleitfähigen Material, wie beispielsweise Teflon ®, zu fertigen.

Die Kontaktflächen 29 des Drehlagers 18 und der Gleitbuchse 27 sind bei der dargestellten Ausführungsform als miteinander korrespondierender Konus und Gegenkonus ausgebildet, um einen festen Sitz der beiden Bauteile aufeinander bzw. ineinander zu gewährleisten. Zur Einstellung des Reibwerts zwischen dem Drehlager 18 und der Gleitbuchse 27 sind das Drehlager 18 und die Gleitbuchse 27 über ein in dem Spannteil 28 der Gleit- und Spannbuchse 26 angeordnetes Spannelement 30 gegeneinander verspannbar, wobei das Spannelement 30 bei der dargestellten Ausführungsform als Madenschraube ausgebildet ist, die zentrisch oder exzentrisch zur Schwenkachse 16 des verschwenkbaren Teilbereichs 14 angeordnet sein kann.

Gemäß einer alternativen Ausführungsform kann das Spannelement 30 auch als als Federelement ausgebildet sein.

Um einerseits ein klemmfreies Aufeinanderschieben des von proximal auf den starren Teilbereich 13 des Griffteils 4 aufschiebbaren verschwenkbaren Teilbereichs 14 des Griffteils 4 zu ermöglichen und andererseits auch eine Zentrierung der Bauteile 13 und 14 in der richtigen Lage zueinander zu ermöglichen, so dass der verschwenkbare Teilbereich 14 des Griffteils 4 auch in der verschwenkten Stellung des Griffteils 4 auf den starren Teilbereich 13 des Griffteils 4 aufschiebbar ist, sind am starren Teilbereich 13 und/oder am verschwenkbaren Teilbereich 14 des Griffteils 4 Anlaufschrägen 31 als Zentrierhilfen ausgebildet.

Die Handhabung des zuvor beschriebenen und in den Abbildungen Fig. 1 bis 4 dargestellten medizinischen Instruments 1 geschieht wie folgt:
In der in Fig. 1 dargestellten Ausgangsstellung, in der die Griffteile 4 und 5 der Handhabe 6 im Wesentlichen horizontal und parallel zueinander ausgerichtet sind, ergreift der Operateur das medizinische Instrument 1, um beispielsweise eine chirurgische Nadel mittels des Werkzeugs 7 zu ergreifen und zu halten. Hierzu drückt der Operateur die mit einer Hand ergriffenen Griffteile 4 und 5 der Handhabe 6 zusammen, bis die Maulteile 7a und 7b geschlossen sind.

Um die Lage der Griffteile 4 und 5 in seiner Hand zu verändern, ohne aber die Handhabe 6 loslassen zu müssen, kann der Operateur den mit den Fingern der Haltehand umgriffenen verschwenkbaren Teilbereich 14 des Griffteils 4 um die Schwenkachse 16 verschwenken, wie dies in Fig. 2 dargestellt ist, wodurch sich auch die Lage des Griffteils 5 an der Handinnenfläche des Operateurs ändert und so zu einer Entlastung der Haltehand führt.

Bei der Darstellung gemäß Fig. 2 wurde der Arretiermechanismus 20 aus Gründen einer besseren Übersichtlichkeit der Abbildung fortgelassen.

Neben der Entlastung der Haltehand bewirkt die Verschwenkbarkeit zumindest eines Teilbereichs 14 des Griffteils 4, dass der Operateur bei jeder Handstellung die Griffteile 4 und 5 der Handhabe 6 immer so zueinander einstellen kann, dass eine bestmögliche Krafteinleitung über die Haltehand auf das medizinische Instrument 1 gewährleistet ist.

Zum Demontieren des verschwenkbaren Teilbereichs 14 des Griffteils 4, beispielsweise zu Reinigungszwecken oder zum Austausch gegen einen anderen verschwenkbaren Teilbereich 14, ist es lediglich erforderlich, den Druckknopf 23 nach unten zu drücken, bis der Rasthaken 22 den Verriegelungsstift 24 wieder freigibt. In dieser eingedrückten Stellung des Druckknopfes 23 kann der verschwenkbare Teilbereich 14 des Griffteils 4 nach proximal vom starren Teilbereich 13 des Griffteils 4 abgezogen werden.

Die Montage des verschwenkbaren Teilbereichs 14 am starren Teilbereich 13 des Griffteils 4 erfolgt in umgekehrter Reihenfolge durch Aufschieben des verschwenkbaren Teilbereichs 14 des Griffteils 4 auf das proximale Ende des starren Teilbereichs 13 des Griffteils 4, bis der Rasthaken 22 des Rastmechanismus 21 den Verriegelungsstift 24 verrastend hintergreift.

Ein solchermaßen ausgebildetes medizinisches Instrument 1 zeichnet sich dadurch aus, dass der verschwenkbare Teilbereich 14 des Grifteils 4 einfach, schnell und ohne Zuhilfenahme eines Werkzeugs vom starren Teilbereich 13 des Griffteils 4 abnehmbar und an diesem wieder festlegbar ist.

### Bezugszeichenliste

- 1: medizinisches Instrument
- 2: Schaft
- 3: Schwenkachse
- 4: Griffteil
- 5: Griffteil
- 6: Handhabe
- 7: Werkzeug
- 7a: starres Maulteil
- 7b: verschwenkbares Maulteil
- 8: Zug-/Druckstange
- 9: Schwenkhebel
- 10: Lagerpunkt
- 11: Schwenkachse
- 12: Federelement
- 13: starrer Teilbereich
- 14: verschwenkbarer Teilbereich
- 15: Längsachse
- 16: Schwenkachse
- 17: Lagerzapfen
- 18: Drehlager
- 19: Griffmulde
- 20: Arretiermechanismus
- 21: Rastmechanismus
- 22: Rasthaken
- 23: Druckknopf
- 24: Verriegelungselement
- 25: Blattfeder
- 26: Gleit- und Spannbuchse
- 27: Gleitbuchse
- 28: Spannteil
- 29: Kontaktfläche
- 30: Spannelement
- 31: Anlaufschräge
- α: Verschwenkwinkel

## Patentansprüche

1. Medizinisches Instrument mit einem Schaft (2), an dessen distalem Ende ein Werkzeug (7) angeordnet ist und an dessen proximalem Ende eine aus mindestens zwei Griffteilen (4, 5) bestehende Handhabe (6) angeordnet ist, wobei das Werkzeug (7) über ein verstellbar ausgebildetes Griffteil (4, 5) der Handhabe (6) betätigbar ist und wobei mindestens ein Griffteil (4) der Handhabe (6) aus einem starren Teilbereich (13) sowie einem um die längsachse des starren Teilbereichs (13) verschwenkbaren Teilbereich (14) besteht,
**dadurch gekennzeichnet,**
**dass** der verschwenkbare Teilbereich (14) des Griffteils (4) über einen Rastmechanismus (21) abnehmbar am starren Teilbereich (13) des Griffteils (4) festlegbar ist und dass der Rastmechanismus (21) einen Rasthaken (22) aufweist der über einen Druckknopf (23) betätigbar ausgebildet ist.

2. Medizinisches Instrument nach Anspruch 1, **dadurch gekennzeichnet, dass** der Rastmechanismus (21) einen an einem der miteinander zu verrastenden Bauteile (13 oder 14) angeordneten, mit einem Rasthaken (22) versehenen federbelasteten Druckknopf (23) und einen an dem anderen Bauteil (14 oder 13) angeordneten, mit dem Rasthaken (22) korrespondierenden Verriegelungselement (24) aufweist.

3. Medizinisches Instrument nach Anspruch 1 oder 2, **dadurch gekennzeichnet, dass** der Rastmechanismus (21) eine am verschwenkbaren Teilbereich (14) festlegbare Gleit-und Spannbuchse (26) umfasst, die als proximales Lager für den verschwenkbaren Teilbereich (14) dient.

4. Medizinisches Instrument nach Anspruch 3, **dadurch gekennzeichnet, dass** der Druckknopf (23) an der Gleit- und Spannbuchse (26) gelagert ist.

5. Medizinisches Instrument nach Anspruch 4, **dadurch gekennzeichnet, dass** die Gleit-und Spannbuchse (26) ein verdrehfest mit dem Druckknopf (23) verbundenes Drehlager (18) sowie eine mit dem Drehlager (18) zusammenwirkende verdrehfest mit dem verschwenkbaren Teilbereich (14) verbundene Gleitbuchse (27) umfasst.

6. Medizinisches Instrument nach Anspruch 4, **dadurch gekennzeichnet, dass** zumindest die Kontaktflächen (29) des Drehlagers (18) und der Gleitbuchse (27) aus einem gleitfähigen Material bestehen.

7. Medizinisches Instrument nach Anspruch 4, **dadurch gekennzeichnet, dass** die Kontaktflächen (29) des Drehlagers (18) und der Gleitbuchse (27) miteinander korrespondierend konisch ausgebildet sind.

8. Medizinisches Instrument nach einem der Ansprüche 5 bis 7 **dadurch gekennzeichnet, dass** das Drehlager (18) und die Gleitbuchse (27) über ein Spannelement (30) gegeneinander verspannbar sind.

9. Medizinisches Instrument nach Anspruch 8, **dadurch gekennzeichnet, dass** das Spannelement (30) als Federelement ausgebildet ist.

10. Medizinisches Instrument nach Anspruch 8, **dadurch gekennzeichnet, dass** das Spannelement (30) als Spannschraube ausgebildet ist.

11. Medizinisches Instrument nach Anspruch 8, **dadurch gekennzeichnet, dass** das Spannelement (30) als Madenschraube ausgebildet ist.

12. Medizinisches Instrument nach einem der Ansprüche 1 bis 11, **dadurch gekennzeichnet, dass** am starren Teilbereich (13) und/oder am verschwenkbaren Teilbereich (14) des Griffteils (4) Anlaufschrägen (31) als Zentrierhilfen ausgebildet sind.

13. Medizinisches Instrument nach einem der Ansprüche 1 bis 11, **dadurch gekennzeichnet, dass** der Verschwenkwinkel (α) des verschwenkbaren Teilbereichs (14) durch die bezüglich der Längsachse (16) des Griffteils (4) exzentrische Lagerung des verschwenkbaren Teilbereichs (14) am starren Teilbereich (13) begrenzt ist.

## Claims

1. Medical instrument with a shaft (2), at the distal end of which a tool (7) is arranged and at the proximal end of which a handle (6) comprising two gripping components (4, 5) is arranged, wherein the tool (7) can be actuated by means of an adjustably formed gripping component (4, 5) of the handle (6) and wherein at least one gripping component (4) of the handle (6) comprises a rigid subregion (13) and a subregion (14) that can pivot about the longitudinal axis of the rigid subregion (13), **characterized in that** the pivotable subregion (14) of the gripping component (4) can be removably fixed on the rigid subregion (13) of the gripping component (4) by means of a latching mechanism (21) and **in that** the latching mechanism (21) has a latching hook (22), which is formed such that it can be operated by means of a pushbutton (23).

2. Medical instrument according to Claim 1, **characterized in that** the latching mechanism (21) has a spring-loaded pushbutton (23), which is arranged on one of the components (13 or 14) to be latched together and is provided with a latching hook (22), and has a locking element (24), which is arranged on the other component (14 or 13) and corresponds to the latching hook (22).

3. Medical instrument according to Claim 1 or 2, **characterized in that** the latching mechanism (21) comprises a sliding and clamping bushing (26), which can be fixed on the pivotable subregion (14) and serves as a proximal bearing for the pivotable subregion (14).

4. Medical instrument according to Claim 3, **characterized in that** the pushbutton (23) is mounted on the sliding and clamping bushing (26).

5. Medical instrument according to Claim 4, **characterized in that** the sliding and clamping bushing (26) comprises a rotary bearing (18), which is connected to the pushbutton (23) in a rotationally fixed manner, and a sliding bushing (27), which interacts with the rotary bearing (18) and is connected to the pivotable subregion (14) in a rotationally fixed manner.

6. Medical instrument according to Claim 4, **characterized in that** at least the contact areas (29) of the rotary bearing (18) and of the sliding bushing (27) consist of a material with sliding properties.

7. Medical instrument according to Claim 4, **characterized in that** the contact areas (29) of the rotary bearing (18) and of the sliding bushing (29) are formed conically in a manner corresponding to one another.

8. Medical instrument according to one of Claims 5 to 7, **characterized in that** the rotary bearing (18) and the sliding bushing (27) can be braced against each other by means of a clamping element (30).

9. Medical instrument according to Claim 8, **characterized in that** the clamping element (30) is formed as a spring element.

10. Medical instrument according to Claim 8, **characterized in that** the clamping element (30) is formed as a clamping screw.

11. Medical instrument according to Claim 8, **characterized in that** the clamping element (30) is formed as a grub screw.

12. Medical instrument according to one of Claims 1 to 11, **characterized in that** run-on slopes (31) are formed as centring aids on the rigid subregion (13) and/or on the pivotable subregion (14) of the gripping component (4).

13. Medical instrument according to one of Claims 1 to 11, **characterized in that** the pivoting angle (α) of the pivotable subregion (14) is limited by the pivotable subregion (14) being mounted on the rigid subregion (13) eccentrically with respect to the longitudinal axis (16) of the gripping component (4).

## Revendications

1. Instrument médical comprenant un bras (2) à l'extrémité distale duquel est disposé un outil (7) et à l'extrémité proximale duquel est disposée une poignée (6) constituée d'au moins deux parties de préhension (4, 5), l'outil (7) pouvant être actionné par le biais d'une partie de préhension (4, 5) de la poignée (6), réalisée de manière déplaçable, et au moins une partie de préhension (4) de la poignée (6) se composant d'une région partielle rigide (13) ainsi que d'une région partielle (14) pouvant pivoter autour de l'axe longitudinal de la région partielle rigide (13), **caractérisé en ce que**
la région partielle pivotante (14) de la partie de préhension (4) pouvant être fixée par le biais d'un mécanisme d'encliquetage (21) de manière amovible sur la région partielle rigide (13) de la partie de préhension (4), et **en ce que** le mécanisme d'encliquetage (21) présente un crochet d'encliquetage (22) qui est réalisé de manière à pouvoir être actionné par le biais d'un bouton poussoir (23).

2. Instrument médical selon la revendication 1, **caractérisé en ce que** le mécanisme d'encliquetage (21) présente un bouton poussoir (23) disposé sur l'un des composants (13 ou 14) à encliqueter l'un à l'autre, sollicité par ressort et pourvu d'un crochet d'encliquetage (22), et un élément de verrouillage (24) correspondant au crochet d'encliquetage (22) et disposé sur l'autre composant (14 ou 13).

3. Instrument médical selon la revendication 1 ou 2, **caractérisé en ce que** le mécanisme d'encliquetage (21) comprend une douille coulissante et de serrage (26) pouvant être fixée sur la région partielle pivotante (14), qui sert de palier proximal pour la région partielle pivotante (14).

4. Instrument médical selon la revendication 3, **caractérisé en ce que** le bouton poussoir (23) est monté sur la douille coulissante et de serrage (26).

5. Instrument médical selon la revendication 4, **caractérisé en ce que** la douille coulissante et de serrage (26) comprend un palier rotatif (18) connecté de manière solidaire en rotation au bouton poussoir (23) ainsi qu'un manchon coulissant (27) connecté de manière solidaire en rotation à la région partielle pivotante (14) et coopérant avec le palier rotatif (18).

6. Instrument médical selon la revendication 4, **caractérisé en ce qu'**au moins les faces de contact (29) du palier rotatif (18) et du manchon coulissant (27) se composent d'un matériau glissant.

7. Instrument médical selon la revendication 4, **caractérisé en ce que** les faces de contact (29) du palier rotatif (18) et du manchon coulissant (27) sont réalisées coniquement de manière à se correspondre mutuellement.

8. Instrument médical selon l'une quelconque des revendications 5 à 7, **caractérisé en ce que** le palier rotatif (18) et le manchon coulissant (27) peuvent être serrés l'un contre l'autre par le biais d'un élément de serrage (30).

9. Instrument médical selon la revendication 8, **caractérisé en ce que** l'élément de serrage (30) est réalisé sous forme d'élément de ressort.

10. Instrument médical selon la revendication 8, **caractérisé en ce que** l'élément de serrage (30) est réalisé sous forme de vis de serrage.

11. Instrument médical selon la revendication 8, **caractérisé en ce que** l'élément de serrage (30) est réalisé sous forme de vis sans tête.

12. Instrument médical selon l'une quelconque des revendications 1 à 11, **caractérisé en ce que** des biseaux d'entrée (31) sont réalisés en tant qu'aide au centrage sur la région partielle rigide (13) et/ou sur la région partielle pivotante (14).

13. Instrument médical selon l'une quelconque des revendications 1 à 11, **caractérisé en ce que** l'angle de pivotement (α) de la région partielle pivotante (14) est limité par le support de la région partielle pivotante (14) sur la région partielle rigide (13), excentré par rapport à l'axe longitudinal (16) de la partie de préhension (4).
